# NEUE EUROPÄISCHE PATENTSCHRIFT

(11) **EP 2 751 073 B2**
(45) Veröffentlichungstag und Bekanntmachung des Hinweises auf die Entscheidung über den Einspruch: **14.12.2022**
(45) Hinweis auf die Patenterteilung: 16.11.2016
(21) Anmeldenummer: 12743169.0
(22) Anmeldetag: 02.08.2012
(51) Int. Cl.: C07C 263/10

(54) **VERFAHREN ZUR HERSTELLUNG VON ISOCYANATEN**
PROCESS FOR PREPARING ISOCYANATES
PROCÉDÉ POUR LA PRÉPARATION D'ISOCYANATES

(30) Priorität: 02.09.2011 EP 11179909
(43) Veröffentlichungstag der Anmeldung: 09.07.2014
(73) Patentinhaber: BASF SE, 67056 Ludwigshafen am Rhein (DE)
(72) Erfinder: MATTKE, Torsten, 67251 Freinsheim (DE); OLBERT, Gerhard, 69221 Dossenheim (DE)
(86) Internationale Anmeldenummer: PCT/EP2012/065176
(87) Internationale Veröffentlichungsnummer: WO 2013/029918

(56) Entgegenhaltungen:
- WO-A1-2010/015667
- WO-A1-2011/003531
- US-A- 3 234 253
- US-A- 3 287 387
- US-A- 3 465 021
- US-A- 4 419 295
- US-A1- 2004 008 572

## Beschreibung

Die Erfindung betrifft ein Verfahren zur Herstellung von Isocyanaten durch Umsetzung der korrespondierenden Amine mit Phosgen, bei dem bei Betrieb unterhalb der Nennkapazität der verwendeten Reaktoranlage die Menge Phosgen erhöht wird und die Menge an zugegebenen inerten Stoffen erhöht wird.

Die Herstellung von Isocyanaten durch Phosgenierung der korrespondierenden Amine kann prinzipiell durch eine Flüssigphasen- oder eine Gasphasen-Phosgenierung erfolgen. Im Unterschied zur Gasphasen-Phosgenierung wird die Reaktion bei der Flüssigphasen-Phosgenierung bei niedrigen Temperaturen durchgeführt, weiterhin ist eine Verdampfung der Edukte nicht erforderlich.

Bei der Flüssigphasenphosgenierung wird ein aminhaltiger Eduktstrom in der Flüssigphase zugeführt. Dieser wird mit einem phosgenhaltigen Eduktstrom vermischt. Hierbei kann das Phosgen in einem inerten Lösungsmittel gelöst sein. Anschließend wird der phosgenhaltige Eduktstrom in einer Mischeinrichtung eingespritzt, in der dieser mit dem aminhaltigen Eduktstrom vermischt. Das Amin und das Phosgen setzen sich unter Freisetzung von HCl zu den entsprechenden Isocyanaten um.

Eine schnelle Vermischung des Amins mit dem Phosgen ist notwendig, da das entstehende Isocyanat bei einer zu niedrigen Phosgenkonzentration mit dem überschüssigen Amin zu Harnstoff bzw. anderen störenden, hochviskosen und festen Nebenprodukten reagiert. Aus diesem Grund sind eine schnelle Vermischung und eine kurze Verweilzeit in der Reaktionskammer erforderlich.

Ein Verfahren zur Flüssigphasen-Phosgenierung von Aminen zur Herstellung von Isocyanaten ist beispielsweise in der WO 2010/015667 A1 beschrieben.

Bei der Gasphasenphosgenierung werden ein aminhaltiger Eduktstrom und ein phosgenhaltiger Eduktstrom jeweils in gasförmigem Zustand vermischt. Das Amin und das Phosgen setzen sich unter Freisetzung von HCl zu den entsprechenden Isocyanaten um. Der aminhaltige Eduktstrom liegt im Allgemeinen in flüssiger Phase vor und muss vor der Vermischung mit dem phosgenhaltigen Strom verdampft und gegebenenfalls überhitzt werden.

Aufgrund des geringen Dampfdrucks, insbesondere der Diamine, erfolgt die Verdampfung bei erhöhter Temperatur. Dadurch werden jedoch auch Zersetzungsreaktionen der Amine bzw. Diamine, beispielsweise Desaminierungen, Demethylierungen und Dimerisierungen hervorgerufen, die sich negativ auf die Selektivität des Gesamtprozesses auswirken.

Zudem setzen bei der Kontaktierung der beiden Eduktströme aufgrund der hohen Temperaturen schnell reaktive Umsetzungen ein. Neben der Phosgenierung des Amins zum Isocyanat können unerwünschte Neben- und Folgereaktionen stattfinden. So kann zum Beispiel bereits gebildetes Isocyanat mit noch nicht abreagiertem Amin einen Harnstoff bilden. Des Weiteren können auch Carbodiimide und Cyanurate entstehen. Dies wirkt sich zum einen auf die Selektivität des Prozesses aus, zum anderen können gebildete feste Nebenprodukte zu Verstopfungen führen und sich so negativ auf die Laufzeit der Anlage auswirken. Daher wird im Allgemeinen angestrebt, die Eduktströme möglichst schnell zu vermischen, um Mischverhältnisse, die die Bildung von Nebenkomponenten beschleunigen, soweit wie möglich zu vermeiden.

Ein Verfahren für die Herstellung von (Poly)isocyanaten in der Gasphase mit optimierter Vermischung der Reaktanden ist beispielsweise in der EP 1 319 655 A2 beschrieben.

Somit sind sowohl bei der Gasphasen-Phosgenierung als auch bei der FlüssigphasenPhosgenierung das Mischen der Edukte sowie die Verweilzeit des Reaktionsgemischs in den entsprechenden Reaktionsräumen kritische Parameter. Die Anlagen zur Herstellung von Isocyanaten durch Phosgenierung von Aminen müssen daher an die speziellen Anforderungen hinsichtlich schneller Durchmischung der Eduktströme und enge Verweilzeitfenster angepasst sein. Anlagen zur Phosgenierung von Aminen werden dabei im Wesentlichen an die maximale Mengenströme bzw. an die jeweilige Nennkapazität ausgelegt. Dies bedeutet, sowohl Mischorgane, wie Düsen, als auch die Reaktionsräume, beispielsweise Verweilzeitreaktoren, arbeiten bei der Nennkapazität im optimalen Bereich mit optimierter Ausbeute, Reinheit der Produkte etc. Ist die Anlage jedoch nicht voll ausgelastet, d.h. sie wird nur bei einem Teil der Nennkapazität betrieben, ändern sich beispielsweise die Verweilzeiten und die Anlage arbeitet nicht mehr im optimalen Bereich. Dies ist beispielsweise bei An- und Abfahrvorgängen, Teilauslastung der Anlage oder Störungen in der Anlage der Fall. In diesen Fällen verminderter Last arbeiten sowohl die Mischorgane als auch die Verweilzeitreaktoren nicht im optimalen Bereich. Die Folge sind Ausbeuteverluste, Foulingprobleme und/oder Qualitätseinbußen.

Aufgabe der vorliegenden Erfindung war daher, ein Verfahren zur Herstellung von Isocyanaten durch Umsetzung der korrespondierenden Amine mit Phosgen bereit zu stellen, das auch bei unterschiedlichen Auslastungen ohne die vorstehend beschriebenen Probleme durchgeführt werden kann, insbesondere soll auch bei Fahren der Anlage im Teillastbereich das Mischen und/oder die Reaktion in dem jeweils optimierten Verweilzeitfenster erfolgen.

Diese Aufgabe wird erfindungsgemäß gelöst durch das folgende Verfahren zur Herstellung von Isocyanaten durch Umsetzung der korrespondierenden Amine, enthalten in mindestens einem Eduktstrom A, mit Phosgen, enthalten in mindestens einem Eduktstrom P, in einer Reaktoranlage umfassend mindestens eine Mischzone und mindestens eine Reaktionszone, wobei Eduktstrom A und/oder Eduktstrom P gegebenenfalls einen oder mehrere inerte Stoffe enthalten, und
wobei in Zeiträumen von mindestens 6 h, in denen der Mengenstrom S^{x} der eingesetzten Amine unterhalb von 95% des Mengenstroms S⁰ der eingesetzten Amine bei Betrieb bei der Nennkapazität der Reaktoranlage liegt, im Vergleich zum Betrieb bei der Nennkapazität des Reaktors
(i) das Verhältnis von Phosgen zu Amin erhöht wird und
(ii) die Konzentration des inerten Stoffes oder der inerten Stoffe in dem Amin enthaltenden Eduktstrom A und/oder dem Phosgen enthaltenden Eduktstrom P erhöht wird.

Das erfindungsgemäße Verfahren erlaubt, eine bestehende Anlage bei unterschiedlichen Auslastungen mit gleichbleibender Produkt- und Verfahrensqualität zu betreiben. Dies kann gegebenenfalls die Anschaffung mehrerer Anlagen mit unterschiedlichen Nennkapazitäten einsparen.

Die Nennkapazität bedeutet dabei die Menge des pro Zeiteinheit produzierten Zielprodukts einer chemischen Anlage, für die die Anlage ausgelegt bzw. dimensioniert wurde. Der Betrieb bei der Nennkapazität gibt daher auch die Mengenströme (Mengen pro Zeiteinheit) vor, die zur Erzielung der Nennkapazität einer Anlage eingesetzt werden. Bei der Herstellung von Isocyanaten aus den entsprechenden Aminen und Phosgen bedeutet das, dass eine Reaktoranlage für die Produktion einer bestimmten Menge Isocyanat in einer bestimmten Zeitspanne ausgelegt ist und dafür bestimmte Mengenströme Amin und Phosgen zugeführt werden müssen.

Im Rahmen der Erfindung bezieht sich der Begriff "unterhalb der Nennkapazität" auf das bzw. die eingesetzten Amine; d.h. "Betrieb unterhalb der Nennkapazität" bedeutet, dass der eingesetzte Mengenstrom Amin kleiner als der Mengenstrom Amin ist, der für den Betrieb bei Nennkapazität einer Reaktoranlage vorgesehen ist. Parameter, die sich auf den Betrieb bei Nennkapazität beziehen, werden vorliegend mit dem Index "0" gekennzeichnet. Parameter, die sich auf den Betrieb unterhalb der Nennkapazität beziehen, sind mit "x" kenntlich gemacht.

Erfindungsgemäß bedeutet der Mengenstrom S° der Amine die der Reaktoranlage zugeführte Menge Amin pro Zeiteinheit, wenn die Reaktoranlage bei ihrer Nennkapazität betrieben wird. Weiterhin bedeutet der Mengenstrom S^{x} der Amine die der Reaktoranlage zugeführte Menge Amin pro Zeiteinheit, wenn die Reaktorkapazität unterhalb ihrer Nennkapazität betrieben wird.

Der Gesamtmengenstrom G° bedeutet die Summe der zugeführten Mengenströme bei Betrieb bei der Nennkapazität der Anlage, berechnet als Summe der Mengenströme Amin(e), Phosgen und gegebenenfalls vorhandener inerter Stoffe. Der Gesamtmengenstrom Gₓ bedeutet die Summe der zugeführten Ströme bei Betrieb unterhalb der Nennkapazität der Anlage, berechnet als Summe der Mengenströme Amin(e), Phosgen und gegebenenfalls vorhandener inerter Stoffe.

Erfindungsgemäß liegt der Mengenstrom S^{x} der Amine zumindest zeitweise unterhalb von 95 % des Mengenstroms S° der Amine, mehr bevorzugt unterhalb von 90 % und besonders bevorzugt unterhalb von 85 % des Mengenstroms S° der Amine.

Die vorliegende Erfindung betrifft ein Verfahren zur Herstellung von Isocyanaten durch Umsetzen von Phosgen mit den entsprechenden Aminen, bei dem eine gegebene Reaktoranlage üblicherweise zumindest zeitweise unterhalb ihrer Nennkapazität betrieben wird.

"Zeitweise" umfasst vorliegend Zeiträume von mindestens 6 h, bevorzugt von mindestens 12 h und besonders bevorzugt von mindestens 24 h. Die Zeiträume, in denen die Reaktoranlage unterhalb ihrer Nennkapazität betrieben werden kann, sind nach oben hin nicht begrenzt, so dass eine Reaktoranlage auch kontinuierlich nach dem erfindungsgemäßen Verfahren unterhalb der Nennkapazität betrieben werden kann, d.h. der Mengenstrom des bzw. der eingesetzten Amine liegt kontinuierlich unterhalb des Mengenstroms der eingesetzten Amine bei Nennkapazität der verwendeten Reaktoranlage. Es ist erfindungsgemäß auch möglich, zwischen Betrieb bei Nennkapazität und Betrieb unterhalb der Nennkapazität bei Anwendung der erfindungsgemäß vorgesehenen Maßnahmen hin und her zu wechseln.

Gemäß des erfindungsgemäßen Verfahrens wird bei Betrieb unterhalb der Nennkapazität der verwendeten Reaktoranlage das Verhältnis von zugeführtem Phosgen zu zugeführtem Amin erhöht (i) und die Konzentration des inerten Stoffes oder der inerten Stoffe im Eduktstrom A und/oder im Eduktstrom P erhöht (ii). Eduktstrom A und/oder Eduktstrom P enthalten gegebenenfalls einen oder mehrere inerte Stoffe, die aus den weiter unten aufgeführten Inertmedien, bevorzugt inerte Lösungsmittel und Inertgase, ausgewählt sind. Falls Eduktstrom A bzw. P bei Betrieb bei Nennkapazität keine inerten Stoffe enthält, bedeutet Erhöhen der Konzentration des inerten Stoffs oder der inerten Stoffe, dass ein oder mehrere inerte Stoffe dem Eduktstrom A und/oder dem Eduktstrom P zugegeben werden, d.h. die Konzentration an inerten Stoffen von 0 auf einen höheren Wert angehoben wird.

Allgemein sind im Rahmen der vorliegenden Erfindung inerte Stoffe, auch Inertmedien genannt, deren Konzentration gemäß (ii) erhöht werden können, solche, die im Reaktionsraum vorliegen und nicht mit den im Reaktionsverlauf auftretenden Verbindungen reagieren. Der oder die inerten Stoffe werden bevorzugt aus inertem Lösungsmittel und Inertgasen ausgewählt. Die inerten Lösungsmittel können je nach Reaktionsführung flüssig oder gasförmig vorliegen. Als Inertmedium können zum Beispiel Stickstoff, Edelgase wie Helium oder Argon, Aromaten wie Chlorbenzol, o-Dichlorbenzol, Trichlorbenzol, Toluol, Xylol, Chlornaphthalin, Decahydronaphtalin, Kohlenstoffdioxid oder Kohlenstoffmonoxid eingesetzt werden. Bevorzugtwerden bei Umsetzung in der Gasphase jedoch Stickstoff und/oder Chlorbenzol als Inertmedium verwendet. Bei der Umsetzung in flüssiger Phase wird bei (ii) bevorzugt die Konzentration des/der dabei üblicherweise eingesetzten Lösungsmittel erhöht.

Gemäß einer Ausführungsform der Erfindung wird der Gesamtmengenstrom G^{x} durch die vorbeschriebenen Maßnahmen (i) und (ii) so angehoben, dass der Gesamtmengenstrom G^{x} bei Betrieb unterhalb der Nennkapazität bei mindestens (x+5) % des Gesamtmengenstroms G° bei Betrieb der Nennkapazität liegt, wobei x % den Prozentsatz des Mengenstroms Amin(e) angibt, auf den der Mengenstrom Amin(e) gegenüber dem Betrieb bei Nennkapazität reduziert ist. Bevorzugt liegt G^{x} bei mindestens (x+8) % des Gesamtmengenstroms G° und besonders bevorzugt liegt G^{x} bei mindestens (x+12) % des Gesamtmengenstroms G°. Wird die Reaktoranlage beispielsweise mit 50 % des Mengenstroms Amin(e), bezogen auf die Nennkapazität, betrieben, so wird G^{x} durch die vorbeschriebenen Maßnahmen (i) und (ii) so angehoben, dass der Gesamtmengenstrom G^{x} bei mindestens 55 %, bevorzugt bei mindestens 58 % und besonders bevorzugt bei mindestens 62 % des Gesamtmengenstroms G⁰ liegt.

Gemäß einerweiteren Ausführungsform der Erfindung wird der Gesamtmengenstrom G^{x} durch die vorbeschriebenen Maßnahmen (i) und (ii) so angehoben, dass er im Bereich von (G°- 15 %) ≤ G^{x} ≤ (G° + 15 %), bevorzugt im Bereich von (G°-10 %) ≤ G^{x} ≤ (G° + 10 %), besonders bevorzugt im Bereich von (G°- 5 %) ≤ G^{x} ≤ (G° + 5 %) und insbesondere im Wesentlich bei G° liegt.

Zur Herstellung des Isocyanats werden der mindestens eine Phosgen enthaltende Eduktstrom P und der mindestens eine Amin enthaltende Eduktstrom A zunächst einer Mischzone zugeführt, in der die Vermischung von Amin enthaltenden Edukstrom A und Phosgen enthaltenden Eduktstrom P zu einem Reaktionsgemisch erfolgt. Dabei muss auf eine ausreichend schnelle Vermischung der Reaktanden geachtet werden. Methoden zur Durchführung kurzer Mischzeiten sind im Prinzip bekannt. In den Mischeinheiten können Mischaggregate mit dynamischen oder statischen Mischern eingesetzt werden. Erfindungsgemäß bevorzugt werden in den Mischeinheiten ein oder mehrere statische Mischorgane eingesetzt. Als statische Mischorgane geeignet sind beispielsweise aus der Verbrennungstechnik bekannte Düsen, Glattstrahldüsen oder Venturidüsen, sowie Lavaldüsen. Eine besonders vorteilhafte Ausführung eines statischen Mischorgans ist in WO2010/015667 A1 beschrieben. Als dynamische Mischer können beispielsweise in den Mischeinheiten angeordnete Rotor/Statorsysteme eingesetzt werden. Erfindungsgemäß bevorzugt werden statische Mischorgane, insbesondere Düsen eingesetzt.

Nach dem Mischen der Eduktströme zu mindestens einem Reaktionsgemisch wird das Reaktionsgemisch in einer Reaktionszone, umfassend die mindestens eine Reaktionseinheit, umgesetzt. Reaktoren, die als Reaktionseinheiten zur Phosgenierung eines Amins zur Herstellung von Isocyanaten eingesetzt werden können, sind dem Fachmann bekannt. Vorzugsweise umfasst eine Reaktionszone jeweils mindestens einen Verweilzeitreaktor. Vorzugsweise werden als Verweilzeitreaktoren Reaktionskolonnen, Rohrreaktoren und/oder Rührkesselkaskaden eingesetzt.

In der Reaktionszone wird das Amin mit dem Phosgen zum korrespondierenden Isocyanat und Chlorwasserstoff umgesetzt. Üblicherweise wird das Phosgen im Überschuss zugegeben, so dass das in der Reaktionszone entstehende Reaktionsgemisch neben dem gebildeten Isocyanat mit dem Chlorwasserstoff auch Phosgen enthält.

Erfindungsgemäß umfasst die Reaktionszone mindestens eine Reaktionseinheit und die Mischzone mindestens eine Mischeinheit.

Als "Einheit" (beispielsweise Mischeinheit, Reaktionseinheit oder Quencheinheit) wird im Rahmen der Erfindung jeweils eine Vorrichtung verstanden, in der der jeweilige Verfahrensschritt (beispielsweise Mischen, Umsetzen oder Quenchen) durchgeführt werden kann. Als Reaktionseinheit kann beispielsweise ein Rohrreaktor eingesetzt werden, als Mischeinheit ein dynamischer Mischer mit Rotor/Statorsystem und als Quencheinheit eine für das Quenchen geeignete Vorrichtung.

Im Anschluss an die Reaktion zu Isocyanat erfolgt die Aufarbeitung des bei der Reaktion erhaltenen Produktgemischs. Zur Aufarbeitung gehören beispielsweise die Abtrennung der gewünschten Isocyanate, die Abtrennung der gegebenenfalls im Produktgemisch enthaltenen Lösungsmittel, Inertgase, Edukte und Quenchmedien, gegebenenfalls durchgeführte Wäschen des Produktgemisches und Kondensationen.

Das erfindungsgemäße Verfahren eignet sich sowohl für die Gasphasen-Phosgenierung als auch für die Flüssigphasen-Phosgenierung.

Gemäß einer Ausführungsform der Erfindung erfolgt die Umsetzung von Amin und Phosgen in der Reaktionszone in der Gasphase. Hierzu liegt der Druck in der Reaktionszone üblicherweise im Bereich zwischen 0,3 bis 3 bar absolut, bevorzugt im Bereich von 0,8 bis 3,0 bar absolut. Die Temperatur liegt dabei üblicherweise im Bereich von 250 bis 550°C, bevorzugt im Bereich von 300 bis 500°C.

Um die Reaktion in der Gasphase durchführen zu können, ist es weiterhin bevorzugt, das Amin und das Phosgen gasförmig zuzugeben. Hierzu weist das Amin vorzugsweise eine Temperatur im Bereich von 200 bis 400°C auf. Der Druck in der Mischzone liegt vorzugsweise in einem Bereich von 0,05 bis 3 bar absolut und die Temperatur in der Mischzone in einem Bereich von 200 bis 400 °C. Die Temperatur in der Mischzone ergibt sich dabei aus der Temperatur des in der Mischzone einströmenden Phosgens und Amins. Die Temperatur des zugegebenen Phosgens liegt vorzugsweise im Bereich von 250 bis 450°C. Hierzu wird das Phosgen üblicherweise vor Zugabe auf dem Fachmann bekannte Weise erwärmt.

Zur Erwärmung des Phosgens und des Amins und zur Verdampfung des Amins wird zum Beispiel eine elektrische Beheizung oder eine direkte oder indirekte Aufheizung durch Verbrennung eines Brennstoffs eingesetzt. Als Brennstoffe werden üblicherweise Brenngase, beispielsweise Erdgas, eingesetzt. Durch die Absenkung der Siedetemperatur durch Absenkung des Drucks des Amins ist jedoch auch eine Beheizung, zum Beispiel durch Wasserdampf, möglich. In Abhängigkeit von der Siedetemperatur des Amins wird hierbei der Druck des Wasserdampfes ausgewählt. Ein geeigneter Dampfdruck des Wasserdampfes liegt zum Beispiel im Bereich von 40 bis 100 bar. Daraus ergibt sich eine Temperatur des Wasserdampfes im Bereich von 250 bis 311 °C. Es ist jedoch auch möglich, Wasserdampf mit einer Temperatur von mehr als 311 °C zur Verdampfung des Amins einzusetzen.

Im Allgemeinen ist es erforderlich, das Amin mehrstufig auf die Reaktionstemperatur zu erwärmen. Im Allgemeinen wird das Amin hierzu zunächst vorgewärmt, dann verdampft und anschließend überhitzt. Im Allgemeinen erfordert die Verdampfung die längsten Verweilzeiten und führt so zu Zersetzungen des Amins. Um dies zu minimieren, ist eine Verdampfung bei niedrigeren Temperaturen, wie es sich zum Beispiel durch den niedrigeren Druck ergibt, vorteilhaft. Um nach der Verdampfung das verdampfte Amin auf Reaktionstemperatur zu überhitzen, ist im Allgemeinen eine Beheizung mit Wasserdampf nicht ausreichend. Zur Überhitzung wird daher üblicherweise eine elektrische Beheizung oder eine direkte oder indirekte Aufheizung durch Verbrennung eines Brennstoffs eingesetzt.

Im Unterschied zur Verdampfung des Amins erfolgt die Verdampfung des Phosgens im Allgemeinen bei deutlich niedrigeren Temperaturen. Aus diesem Grund kann zur Verdampfung des Phosgens im Allgemeinen Wasserdampf eingesetzt werden. Jedoch ist auch die notwendige Überhitzung des Phosgens, um dieses auf Reaktionstemperatur zu erwärmen, im Allgemeinen nur durch eine elektrische Beheizung oder eine direkte oder indirekte Aufheizung durch Verbrennung eines Brennstoffes möglich.

Bevorzugt wird der mindestens eine Amin enthaltende Eduktstrom A und der mindestens eine Phosgen enthaltende Eduktstrom P jeweils in mindestens einer Verdampfungszone in die gasförmige Phase überführt und gegebenenfalls in mindestens einer Überhitzungszone weiter überhitzt.

Eine Verdampfungszone umfasst mindestens eine Verdampfungseinheit, eine Überhitzungszone umfasst mindestens eine Überhitzungseinheit.

Gleiches gilt für den mindestens einen Phosgen enthaltenden Eduktstrom P. Bevorzugt werden sowohl der mindestens eine Amin enthaltende Eduktstrom A sowie der mindestens eine Phosgen enthaltende Eduktstrom P jeweils in mindestens einer Verdampfungszone in die gasförmige Phase überführt und in mindestens einer Überhitzungszone überhitzt.

Die Umsetzung in der Gasphase kann in Gegenwart mindestens eines Inertmediums durchgeführt werden. Das Inertmedium kann dabei dem Phosgen enthaltenden Eduktstrom und/oder dem Amin enthaltenden Eduktstrom zugegeben werden.

Im Allgemeinen wird das Inertmedium in einer Menge zugesetzt, so dass das Verhältnis der Gasvolumina von Inertmedium zu Amin beziehungsweise zu Phosgen weniger als 0,0001 bis 30, bevorzugt weniger als 0,01 bis 15 und besonders bevorzugt weniger als 0,1 bis 5 beträgt.

Um die Bildung von Nebenprodukten zu vermeiden, ist es bevorzugt, Phosgen im Überschuss zuzuführen. Um nur den für die Reaktion notwendigen Anteil an Aminen zuzuführen, ist es möglich, das Amin mit einem Inertgas zu mischen. Durch den Anteil an Inertgas im Amin lässt sich die Menge des zugeführten Amins bei vorgegebener Geometrie der Zufuhröffnungen für das Amin und das Phosgen einstellen.

Bei der Gasphasenphosgenierung ist es anzustreben, dass die im Reaktionsverlauf auftretenden Verbindungen, also Edukte (Amin und Phosgen), Zwischenprodukte (insbesondere die intermediär entstehenden Mono- und Dicarbamoylchloride), Endprodukte (Diisocyanat), sowie gegebenenfalls zudosierte inerte Verbindungen, unter den Reaktionsbedingungen in der Gasphase verbleiben. Sollten sich diese oder andere Komponenten aus der Gasphase zum Beispiel an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere bei Auftreten der Aminhydrochloride, die sich aus freien Aminogruppen und Chlorwasserstoff bilden, da die resultierenden Aminhydrochloride leicht ausfallen und nur schwer wieder verdampfbar sind.

Um die Bildung unerwünschter Nebenprodukte zu reduzieren beziehungsweise zu vermeiden und weiterhin auch eine Zersetzung des gebildeten Isocyanats zu unterbinden, wird das Reaktionsgas vorzugsweise unmittelbar nach der Reaktion in einem Quench abgekühlt. Hierzu wird ein vorzugsweise flüssiges Quenchmedium zugegeben. Durch Verdampfung des Quenchmediums nimmt dieses Wärme auf und führt zu einer schnellen Abkühlung des Reaktionsgases.

Gemäß einer bevorzugten Ausführungsform des Verfahrens wird bei der Gasphasen-Phosgenierung das erhaltene Produktgemisch in mindestens einer Quenchzone abgekühlt. Das Quenchen wird in einer Quenchzone durchgeführt, die aus mindestens einer Quencheinheit aufgebaut ist.

Eine schnelle Abkühlung wird insbesondere dadurch erzielt, dass das Quenchmedium fein zerstäubt zugegeben wird. Hierdurch weist das Quenchmedium eine große Oberfläche auf und kann schnell die Wärme aufnehmen und damit das Reaktionsgas abkühlen.

Insbesondere bei Verwendung eines Quenchmediums, das bei den Bedingungen im Quenchraum eine Siedetemperatur unterhalb der Kondensationstemperatur des Reaktionsgases aufweist, ist der Druck in den Zuleitungen gegenüber dem Druck im Quenchraum erhöht, um ein Verdampfen des Quenchmediums vor der Zugabe in den Quenchraum zu vermeiden.

Der Druck, mit dem das Quenchmedium zugegeben wird, liegt vorzugsweise im Bereich von 1 bis 20 bar, mehr bevorzugt im Bereich von 1 bis 10 bar und insbesondere im Bereich von 1 bis 8 bar.

Das zur Kühlung eingesetzte Quenchmedium enthält vorzugsweise ein Lösungsmittel, das ausgewählt ist aus der Gruppe bestehend aus Monochlorbenzol, Dichlorbenzol, Trichlorbenzol, Hexan, Benzol, 1,3,5-Trimethylbenzol, Nitrobenzol, Anisol, Chlortoluol, o-Dichlorbenzol, Diethylisophthalat, Tetrahydrofuran, Dimethylformamid, Xylol, Chlornaphthalin, Decahydronaphthalin und Toluol.

Bevorzugt enthält das Quenchmedium einen Teil des im Quench abgekühlten Produktstroms, besonders bevorzugt wird als Quenchmedium ein Teil des bereits im Quench abgekühlten Produktstroms eingesetzt. In diesem Fall enthält das Quenchmedium üblicherweise kein Lösungsmittel sonders lediglich die beim Quenchen auskondensierten Teile des Produktstroms.

Um zu vermeiden, dass sich in Rohrleitungen, Regeleinrichtungen und sonstigen Apparateteilen, insbesondere in den Zerstäuberdüsen des Quenchs Ablagerungen bilden, werden gegebenenfalls im Quenchmedium enthaltene Feststoffpartikel vor Zugabe in den Quench entfernt.

Wenn ein Isocyanat im Quenchmedium enthalten ist, so ist es insbesondere bevorzugt, wenn das bei der Reaktion gebildete Isocyanat zunächst im Quench und gegebenenfalls in nachfolgenden Kühlstufen abgekühlt wird und nach der Abkühlung ein Teilstrom als Quenchmedium eingesetzt wird.

Es ist bevorzugt, wenn das Quenchmedium flüssig zugegeben wird, um eine schnelle Abkühlung des Reaktionsgases im Quench zu erzielen. Die Temperatur des Quenchmediums liegt dabei vorzugsweise im Bereich von 0 bis 250°C, insbesondere im Bereich von 20 bis 220°C. Durch das Einbringen des Quenchmediums in das heiße Reaktionsgas wird das Quenchmedium erwärmt und/oder verdampft. Die für das Erwärmen und die Verdampfung des Quenchmediums notwendige Wärme wird dem Reaktionsgas entzogen und das Reaktionsgas wird auf diese Weise abgekühlt. Die Temperatur, auf die das Reaktionsgas abgekühlt wird, lässt sich zum Beispiel durch die Menge und die Temperatur des zugegebenen Quenchmediums einstellen.

Um die Temperatur, mit der das Quenchmedium in den Quench zugegeben wird, falls erforderlich einzustellen, wird das Quenchmedium vorzugsweise durch einen Wärmetauscher geleitet. Je nach Eintrittstemperatur des Quenchmediums in dem Wärmetauscher kann das Quenchmedium in diesem erwärmt oder abgekühlt werden. Ein Abkühlen ist zum Beispiel dann erforderlich, wenn ein Teil des Produktstromes, der als Quenchmedium eingesetzt wird, direkt im Anschluss an den Quench entnommen wird. Ein Erwärmen kann sich zum Beispiel dann ergeben, wenn der Teil des Produktstromes, der als Quenchmedium eingesetzt wird, am Ende der Aufbereitungsstrecke entnommen wird und eine Temperatur aufweist, die niedriger ist als die gewünschte Temperatur, mit der das Quenchmedium in den Quench zugegeben werden soll. Im Allgemeinen wird es jedoch erforderlich sein, das Quenchmedium vor Zugabe in den Quench abzukühlen.

Wenn das Quenchmedium Lösungsmittel enthält, ist es bevorzugt, dem Quenchmedium vor Zugabe in den Quench das/die Lösungsmittel zuzugeben. Hierdurch können Lösungsmittelverluste im Quenchmedium ausgeglichen werden. Geeignete Lösungsmittel, die im Quenchmedium enthalten sein können, sind zum Beispiel gegebenenfalls mit Halogen substituierte Kohlenwasserstoffe. Vorzugsweise ist das im Quenchmedium enthaltene Lösungsmittel ausgewählt aus der Gruppe bestehend aus Monochlorbenzol, Dichlorbenzol, Trichlorbenzol, Hexan, Benzol, 1,3,5-Trimethylbenzol, Nitrobenzol, Anisol, Chlortoluol, o-Dichlorbenzol, Diethylisophthalat, Dimethylisophthalat, Tetrahydroforan, Dimethylformamid, Xylol, Chlornaphthalin, Decahydronaphtalin und Toluol.

In einer bevorzugten Ausführungsform schließen sich zur weiteren Behandlung an den Quench weitere Stufen zur Abkühlung des Reaktionsgases an. In den einzelnen Stufen zur Abkühlung erfolgt dabei jeweils eine weitere Abkühlung des Produktstromes bis zum Erreichen der gewünschten Temperatur, mit der der Produktstrom beispielsweise einer nachfolgenden Aufarbeitung zugeführt wird. Als Produktstrom wird im Quench dabei vorzugsweise der gesamte, den Quench verlassende Strom eingesetzt, der sowohl das Quenchmedium, als auch das Reaktionsgemisch enthält.

Die weiteren Stufen zur Abkühlung, die sich an den Quench anschließen können, können zum Beispiel weitere Quenche oder Kondensatoren oder beliebige andere Stufen zur Abkühlung, die dem Fachmann bekannt sind, sein. Bevorzugt ist mindestens eine der sich an den Quench anschließenden Stufen zur Abkühlung des Produktstromes ein Kondensator. Als Kondensator eignet sich dabei jede beliebige, dem Fachmann bekannte Kondensatorbauart. Üblicherweise wird als Kondensator ein Wärmetauscher eingesetzt, der von einem Kühlmedium durchströmt wird. Als Kühlmittel kann zum Beispiel Wasser eingesetzt werden. In diesem Fall kondensiert das Gas zumindest teilweise an den Wandungen des Kondensators aus. Die so entstehende Flüssigkeit läuft ab und wird gesammelt und dem Kondensator entnommen.

Nach dem Kondensieren des Produktstromes wird im Allgemeinen eine Aufbereitung angeschlossen. So ist es zum Beispiel möglich, dass das auskondensierte Gemisch in einem Lösungsmittel gewaschen wird. Als Lösungsmittel können zum Beispiel die gleichen Stoffe eingesetzt werden, die auch als Quenchmedium eingesetzt werden können.

Weiterhin ist es zum Beispiel möglich, das den Quench und die sich gegebenenfalls daran anschließenden Stufen zur Abkühlung verlassende Reaktionsgas vorzugsweise bei Temperaturen von mehr als 130°C mit einem Lösungsmittel zu waschen. Als Lösungsmittel eignen sich zum Beispiel die gleichen Stoffe, die auch als Quenchmedium eingesetzt werden können.

Alternativ zur Abkühlung des Produktstromes ist es auch möglich, dass der Produktstrom nach Austritt aus dem Quench einer Trennstufe zugeführt wird. Eine entsprechende Trennstufe kann sich alternativ jedoch auch zum Beispiel dem Kondensator anschließen. Bevorzugt schließt sich jedoch die Trennstufe direkt an den Quench an. Als Trennstufen eignen sich zum Beispiel Destillationskolonnen oder Wäscher.

Wenn die Trennstufe ein Wäscher ist, so wird der den Quench verlassende Produktstrom vorzugsweise mit einem Lösungsmittel gewaschen. Hierbei wird das Isocyanat selektiv in die Waschlösung überführt. Anschließend werden das verbleibende Gas und die erhaltene Waschlösung bevorzugt mittels Rektifikation in Isocyanat, Lösungsmittel, Phosgen und Chlorwasserstoff aufgetrennt. Als Wäscher eignet sich insbesondere ein Waschturm, in dem aus dem gasförmigen Produktstrom das gebildete Isocyanat durch Kondensation in einem inerten Lösungsmittel abgetrennt wird, während überschüssiges Phosgen, Chlorwasserstoff und gegebenenfalls das Inertmedium den Waschturm gasförmig durchlaufen. Bevorzugt wird dabei die Temperatur des inerten Lösungsmittels oberhalb der Lösungstemperatur des zum Amin gehörigen Carbamoylchlorids im gewählten Waschmedium gehalten. Besonders bevorzugt wird dabei die Temperatur des inerten Lösungsmittels oberhalb der Schmelztemperatur des zum Amin gehörigen Carbamoylchlorids gehalten.

Als Wäscher eignet sich jeder beliebige, dem Fachmann bekannte Wäscher. So können zum Beispiel Rührbehälter oder andere herkömmliche Apparaturen, beispielsweise Kolonnen oder Mixer-Settler-Apparaturen eingesetzt werden.

Das Waschen und die Aufarbeitung des den Quench verlassenen Gemischs aus Reaktionsgas und Quenchmedium erfolgt dabei im Allgemeinen wie beispielsweise in WO-A 2007/028715 beschrieben.

Wenn die Trennstufe eine Destillationskolonne, auch Rektifikationskolonne genannt, ist, so wird der gasförmige Produktstrom der Rektifikationskolonne zugeführt. Die Rektifikationskolonne wird vorzugsweise so betrieben, dass die Temperatur am Kopf der Rektifikationskolonne niedriger ist als die Siedetemperatur des Produktstromes. Auf diese Weise kondensieren in der Destillationskolonne selektiv einzelne Bestandteile des Produktstromes aus und können der Kolonne am Sumpf, über Kopf und gegebenenfalls über Seitenabzüge entnommen werden.

Wenn zur Aufbereitung des Produktstromes ein Kondensator eingesetzt wird, ist es bevorzugt, das Quenchmedium dem Kondensator zu entnehmen. Bei einer Aufbereitung durch Rektifikation erfolgt vorzugsweise eine Abtrennung des als Quenchmedium eingesetzten Lösungsmittels. Das Lösungsmittel enthält in diesem Fall noch Anteile an Isocyanaten. Das so abgetrennte Gemisch aus Lösungsmittel und Isocyanat wird dann als Quenchmedium eingesetzt.

Wenn ein Teil des Produktstromes als Quenchmedium eingesetzt wird, so ist es möglich, diesen Teil zum Beispiel nach dem Abkühlen aus dem Produktstrom abzuzweigen. Alternativ kann das Quenchmedium auch nach einer sich an den Quench anschließenden Aufarbeitung aus einem beliebigen Strom abgezweigt werden.

Gemäß einer weiteren Ausführungsform der Erfindung erfolgt die Umsetzung in flüssiger Phase. Diese Ausführungsform wird nachstehend genauer beschreiben. Erfindungsgemäß bevorzugt liegt in dem mindestens einen Amin enthaltenden Eduktstrom das Amin als Lösung oder als Suspension des entsprechenden Hydrochlorids vor.

Als Eduktströme für das erfindungsgemäße Verfahren mit Flüssigphasen-Phosgenierung werden üblicherweise einerseits 3 gew.-%ige bis 100 gew.-%ige, vorzugsweise 50 gew.-%ige bis 100 gew.-%ige Phosgenlösungen, und andererseits 5 gew.-%ige bis 95 gew.-%ige Lösungen bzw. Suspensionen von Aminen bzw. deren Salzen in geeigneten Lösungsmitteln eingesetzt.

Geeignete Lösungsmittel zur Herstellung der Phosgen- und Aminlösungen bzw. Suspensionen sind beliebige, unter den Reaktionsbedingungen inerte Lösungsmittel wie beispielsweise Monochlorbenzol, o-Dichlorbenzol, Trichlorbenzol, Toluol, Xylol, Methylenchlorid, Perchlorethylen, Trichlorfluoromethan, Butylacetat, Hexan, Heptan, Octan, Biphenyl, Essigsäureethylester, 1,2-Diacetoxyethan, 2-Butanon, Acetonitril und Sulfan. Beliebige Gemische der beispielhaft genannten Lösungsmittel können selbstverständlich auch eingesetzt werden. Zweckmäßigerweise wird man für die Aminkomponente und das Phosgen das gleiche Lösungsmittel bzw. Lösungsmittelgemisch einsetzen, obwohl dies im Rahmen der Erfindung nicht zwingend erforderlich ist.

Die Zuführung der Edukte wird gemäß einer bevorzugten Ausführungsform der Erfindung so eingestellt und/oder geregelt, dass die Phosgen-und Aminlösungen bzw. Aminsuspensionen in solchen Mengen in die Mischkammer geleitet werden, dass in dieser ein Molverhältnis Phosgen zu primären Aminogruppen von ca. 15:1 bis 1:1, bevorzugt 10:1 bis 2:1 vorliegt.

Gemäß einer bevorzugten Ausführungsform ist die Lösung von Phosgen, also der phosgenhaltige Eduktstrom P, frei von Isocyanaten. Dies bedeutet, dass in dem phosgenhaltigen Eduktstrom P Isocyanate in einer Menge von kleiner oder gleich 5 Gew.-%, vorzugsweise kleiner 2 Gew.-%, insbesondere kleiner 1 Gew.-% vorhanden sind. Es ist besonders bevorzugt, dass in dem phosgenhaltigen Eduktstrom P keine Isocyanate enthalten sind, also diese mit den üblichen Analysemethoden nicht nachgewiesen werden können. Vorteilhafterweise lässt sich hierdurch die Bildung von Reaktionsnebenprodukten wie Harnstoffderivaten, welche sich negativ auf die Selektivität des Prozesses auswirkt und zur Verschmutzung der Anlage bis hin zur Verblockung führen kann, deutlich verringern. Die Bildung von Harnstoffderivaten wird somit verringert, indem dem Verfahren keine oder im Wesentlichen keine Isocyanate, die in Kontakt mit Aminen zur Bildung von Harnstoffderivaten führen können, als Edukte zugeführt werden.

Bei der Durchführung des Verfahrens mit Flüssigphasen-Phosgenierung wird die Temperatur in der Mischzone üblicherweise auf einer Temperatur oberhalb der Zersetzungstemperatur des Carbamoylchlorids des verwendeten Amins gehalten. Für die meisten Amine wird das erfindungsgemäße Verfahren bei einer Temperatur von etwa 30 °C bis 300 °C, bevorzugt von etwa 40 °C bis 150 °C, besonders bevorzugt von etwa 50 °C bis 120 °C durchgeführt.

Bei der erfindungsgemäßen Phosgenierung in der Flüssigphase wird mindestens ein Produktgemisch erhalten, das üblicherweise direkt der Aufarbeitung zugeführt wird und dabei teilweise in HCl, Phosgen, Lösungsmittel sowie gebildete Produkte und Nebenprodukte aufgetrennt wird.

Generell können die dem Fachmann zur Herstellung von Isocyanaten bekannten Amine in dem erfindungsgemäßen Verfahren eingesetzt werden. Dabei handelt es sich beispielsweise um Monoamine, Diamine, Triamine und höherwertige Amine. Bevorzugt werden Monoamine und Diamine eingesetzt, besonders bevorzugt Diamine. Entsprechend der eingesetzten Amine ergeben sich die korrespondierenden Monoisocyanate, Diisocyanate, Triisocyanate oder höherwertigen Isocyanate. Bevorzugt werden Monoisocyanate oder Diisocyanate mit dem erfindungsgemäßen Verfahren hergestellt.

Amine und Isocyanate können aliphatisch, cycloaliphatisch oder aromatisch sein. Bevorzugt sind die Amine aliphatisch oder cycloaliphatisch, besonders bevorzugt aliphatisch.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

Im Folgenden wird die Bezeichnung (cyclo)aliphatische Isocyanate für cycloaliphatische und/oder aliphatische Isocyanate verwendet.

Beispiele für aromatische Mono- und Diisocyanate sind bevorzugt solche mit 6 bis 20 C-Atomen, beispielsweise Phenylisocyanat, monomeres 2,4'- und/oder 4,4'-Methylen-di(phenylisocyanat) (MDI) sowie dessen höhere Oligomere (Polymehylen(phenalisocyanat) PDMI) und Mischungen daraus, 2,4- und/oder 2,6-Toluylendiisocyanat (TDI) und 1,5- oder 1,8-Naphthyldiisocyanat (NDI).

Beispiele für (cyclo)aliphatische Diisocyanate sind aliphatische Diisocyanate wie 1,4-Tetramethylendiisocyanat, 1,6-Hexamethylendiisocyanat (1,6-Diisocyanatohexan), 1,8-Octamethylendiisocyanat, 1,10-Decamethylendiisocyanat, 1,12-Dodecamethylendiisocyanat, 1,14-Tetradecamethylendiisocyanat, 1,5-Diisocyanatopentan, Neopentandiisocyanat, 2-Methyl-1,5-düsocyanatopentan, Derivate des Lysindiisocyanats, Tetramethylxylylendüsocyanat (TMXDI), Trimethylhexandiisocyanat oder Tetramethylhexandiisocyanat, sowie 3(beziehungsweise 4)-8(beziehungsweise 9)-Bis(isocyanatomethyl)-tricyclo-[5.2.1.0²⁻⁶]-decan-Isomerengemische, sowie cycloaliphatische Diisocyanate wie 1,4-, 1,3- oder 1,2-Diisocyanato-cyclohexan, 4,4'- oder 2,4'-Di(isocyanatocyclohexyl)methan, 1-Isocyanato-3,3,5-tri-methyl-5-(isocyanatomethyl)cyclohexan (Isophorondiisocyanat), 1,3- oder 1,4-Bis(isocyanatomethyl)cyclohexan, 2,4-oder 2,6-Diisocyanato-1 -methylcyclohexan.

Bevorzugte (cyclo)aliphatische Diisocyanate sind 1,6-Diisocyanatohexan, 1-Isocyanato-3,3,5-trimethyl-5-(isocyanatomethyl)cyclohexan und 4,4'-Di(isocyanatocyclohexyl)-methan. Besonders bevorzugt sind 1,6-Diisocyanatohexan, 1,5-Diisocyanatopentan, 1-Isocyanato-3,3,5-trimethyl-5-(isocyanatomethyl)cyclohexan und 4,4'-Di(isocyanatocyclohexyl)methan.

Geeignete Amine, die bei dem erfindungsgemäßen Verfahren mit GasphasenPhosgenierung zur Reaktion zu den korrespondierenden Isocyanaten eingesetzt werden können, sind solche, bei denen das Amin, die korrespondierenden Zwischenprodukte und die korrespondierenden Isocyanate bei den gewählten Reaktionsbedingungen gasförmig vorliegen. Bevorzugt sind Amine, die sich während der Dauer der Reaktion unter den Reaktionsbedingungen zu höchstens 2 Mol-%, besonders bevorzugt zu höchstens 1 Mol-% und ganz besonders bevorzugt zu höchstens 0,5 Mol-% zersetzen. Besonders geeignet sind hier Amine, insbesondere Diamine, auf Basis von aliphatischen oder cycloaliphatischen Kohlenwasserstoffen mit 2 bis 18 Kohlenstoffatomen. Beispiele hierfür sind 1,6-Diaminohexan, 1,5-Diaminopentan, 1,3-Bis(aminomethyl)cyclohexan, 1-Amino-3,3,5-trimethyl-5-aminomethyl-cyclohexan (IPDA) und 4,4-Diaminodicyclohexylmethan. Bevorzugt verwendet wird 1,6-Diaminohexan (HDA).

Ebenfalls können für das erfindungsgemäße Verfahren mit Gasphasen-Phosgenierung aromatische Amine verwendet werden, die ohne signifikante Zersetzung in die Gasphase überführt werden können. Beispiele für bevorzugte aromatische Amine sind Toluylendiamin (TDA), als 2,4- oder 2,6-Isomer oder als Gemisch davon, beispielsweise als 80:20 bis 65:35 (Mol/Mol)-Gemisch, Diaminobenzol, 2,6-Xylidin, Naphthyldiamin (NDA) und 2,4'-, 2,2'- oder 4,4'-Methylen(diphenyldiamin) (MDA) oder Isomerengemische davon. Bevorzugt sind unter diesen die Diamine, besonders bevorzugt sind 2,4- und/oder 2,6-TDA sowie 2,4'- und/oder 4,4'-MDA.

Besonders bevorzugt für die Gasphasen-Phosgenierung nach dem erfindungsgemäßen Verfahren ist das Amin ausgewählt aus der Gruppe bestehend aus 1,6-Diaminohexan, monomeres 2,4'-Methylen(diphenylamin), monomeres 4,4'-Methylen(diphenylamin), 2,4-Toluylendiamin, 2,6-Toluylendiamin, 1-Amino-3,3,5-trimethyl-5-Aminomethylcyclohexan und Mischungen davon.

Für das erfindungsgemäße Verfahren mit Flüssigphasen-Phosgenierung besonders geeignete Amine sind beliebige primäre Mono- und Polyamine wie z.B. Methylamin, Ethylamin, Butylamin, Stearylamin, Phenylamin, p-Tolylamin, 1,4-Diaminobutan, 1,6-Diaminohexan, 1,8-Diaminooctan, 1,4-Diaminobenzol, 2,4-Diaminotoluol, 2,6-Diaminotoluol, Gemische der beiden letztgenannten Isomeren, 2,2'-Diaminodiphenylmethan, 2,4'-Diaminodiphenylmethan, 4,4'-Diaminodiphenylmethan, Gemische der drei letztgenannten Isomeren, Alkyl-substituierte Diamine der Diphenylmethanreihe, wie beispielsweise 3,4'-Diamino-4-methyldiphenylmethan, Polyamingemische der Diphenylmethanreihe, wie sie in an sich bekannter Weise durch Anilin-Formaldehyd-Kondensationen erhalten werden, p-Xylendiamin, perhydriertes 2,4- und/oder 2,6-Diaminotoluol, 2,2'-, 2,4'- und/oder 4,4'-Diaminodicyclohexylmethan, 1-Amino-3,3,5-trimethyl-5-aminomethyl-cyclohexan (Isophorondiamin, abgekürzt IPDA), Lysin-ethylester, Lysin-aminoethylester, 2,4- und 2,6-Toluydendiamin und 1,6,11-Triamino-undecan.

Im Folgenden wird die Erfindung anhand von Beispielen erläutert, die aus thermodynamischen und kinetischen Daten berechnet wurden.

### Vergleichsbeispiel 1:

In einer Anlage zur Herstellung von Toluylendiisocyanat (TDI) durch Phosgenierung von TDA in der Flüssigphase werden bei Betrieb bei Nennkapazität eine 30 gew.-%ige Lösung von Toluylendiamin (TDA) in Monochlorbenzol mit einem Phosgenstrom in einer Mischdüse (Mischzeit 11,7 ms) vermischt. Das molare Verhältnis von Phosgen zu TDA beträgt 10. Die Mischtemperatur der Ströme beträgt etwa 60 °C. Stromab der Mischdüse ist ein adiabater Rohrreaktor mit einer Verweilzeit von etwa 2 min installiert. In diesem erfolgt die Umsetzung des Amins zu Isocyanat bzw. zum Carbamylchlorid als Vorstufe. Durch adiabate Temperaturerhöhung sowie durch Freisetzung von HCl wird eine Gasphase ausgebildet. Das zweiphasige Reaktionsgemisch wird anschließend einer Reaktionskolonne zugeführt.

Die Ausbeute der Reaktionsstufe bezüglich TDA beträgt 93,4 %.

### Vergleichsbeispiel 2 :

Die oben beschriebene Anlage soll nur 50 % ihrer Nennlast produzieren. Entsprechend werden aminhaltiger und phosgenhaltiger Eduktstrom halbiert. Infolgedessen steigt die Mischzeit auf 23,4 ms. Gleichzeitig steigt die Verweilzeit im Reaktor auf das Doppelte. Die Ausbeute an TDI fällt von 93,4 % auf 80,2 %

Die oben beschriebene Anlage wird wie in Vergleichsbeispiel 2 bei 50 % ihrer Nennkapazitätbetrieben, jedoch wird die Menge des inerten Lösungsmittels Monochlorbenzol erhöht, so dass die Konzentration von Toluyldiamin in Monochlorbenzol 18 Gew.-% beträgt. Die Gesamtstrommenge G^{x}, berechnet als Summe der Mengenströme Amin(e), Phosgen und inertem Lösungsmittel liegt bei 64 % der entsprechenden Gesamtstrommenge G° aus Vergleichsbeispiel 1 bei Betrieb bei Nennkapazität. Die Mischzeit beträgt ca. 20,9 ms. Durch die stärkere Verdünnung des TDA kann die Ausbeute jedoch wieder auf 93,4 % gesteigert werden.

## Patentansprüche

1. Verfahren zur Herstellung von Isocyanaten durch Umsetzung der korrespondierenden Amine, enthalten in mindestens einem Eduktstrom A, mit Phosgen, enthalten in mindestens einem Eduktstrom P, in einer Reaktoranlage umfassend mindestens eine Mischzone und mindestens eine Reaktionszone, wobei Eduktstrom A und/oder Eduktstrom P gegebenenfalls einen oder mehrere inerte Stoffe enthalten, und
wobei in Zeiträumen von mindestens 6 h, in denen der Mengenstrom S^{x} der eingesetzten Amine unterhalb von 95 % des Mengenstroms S⁰ der eingesetzten Amine bei Betrieb bei der Nennkapazität der Reaktoranlage liegt, im Vergleich zum Betrieb bei der Nennkapazität des Reaktors
(i) das Verhältnis von Phosgen zu Amin erhöht wird und
(ii) die Konzentration des inerten Stoffs oder der inerten Stoffe in dem Amin enthaltenden Eduktstrom A und/oder dem Phosgen enthaltenden Eduktstrom P erhöht wird.

2. Verfahren nach Anspruch 1, **dadurch gekennzeichnet, dass** der Mengenstrom Amin auf x% gegenüber der Nennkapazität reduziert ist und der Gesamtmengenstrom G^{x} aus Amin, Phosgen und gegebenenfalls vorhandenen inerten Stoffen mindestens (x+5) % des Gesamtmengenstroms G⁰ aus Amin, Phosgen und gegebenenfalls vorhandenen inerten Stoffen bei Nennkapazität der Anlage ist.

3. Verfahren nach Anspruch 1 oder 2, **dadurch gekennzeichnet, dass** der oder die inerten Stoffe ausgewählt sind aus inerten Lösungsmitteln und Inertgasen.

4. Verfahren nach einem der Ansprüche 1 bis 3, **dadurch gekennzeichnet, dass** die Umsetzung in flüssiger Phase erfolgt.

5. Verfahren nach Anspruch 4, **dadurch gekennzeichnet, dass** in dem mindestens einen Amin enthaltenden Eduktstrom das Amin als Lösung oder als Suspension des entsprechenden Hydrochlorids in einem inerten Lösungsmittel vorliegt.

6. Verfahren nach Anspruch 4 oder 5, **dadurch gekennzeichnet, dass** das Amin ausgewählt ist aus der Gruppe bestehend aus Methylamin, Ethylamin, Butylamin, Stearylamin, Phenylamin, p-Tolylamin, 1,4-Diaminobutan, 1,6-Diaminohexan, 1,8-Diaminooctan, 1,4-Diaminobenzol, 2,4- und/oder 2,6-Diaminotoluol, 2,2'-, 2,4'- und/oder 4,4'-Diaminodiphenylmethan, Alkyl-substituierte Diamine der Diphenylmethanreihe, wie beispielsweise 3,4'-Diamino-4-methyldiphenylmethan, Polyamingemische der Diphenylmethanreihe, wie sie in an sich bekannter Weise durch Anilin-Formaldehyd-Kondensationen erhalten werden, p-Xylendiamin, perhydriertes 2,4- und/oder 2,6-Diaminotoluol, 2,2'-, 2,4'- und/oder 4,4'-Diaminodicyclohexylmethan, 1-Amino-3,3,5-trimethyl-5-aminomethylcyclohexan (Isophorondiamin, abgekürzt IPDA), Lysinethylester, Lysinaminoethylester, 2,4- und/oder 2,6-Toluydendiamin, 1,6,11-Triaminoundecan und Mischungen davon.

7. Verfahren nach einem der Ansprüche 4 bis 6, **dadurch gekennzeichnet, dass** als Lösungsmittel für die Edukte Monochlorbenzol, o-Dichlorbenzol, Trichlorbenzol, Toluol, Xylol, Methylenchlorid, Perchlorethylen, Trichlorfluoromethan, Butylacetat, Hexan, Heptan, Octan, Biphenyl, Essigsäureethylester, 1,2-Diacetoxyethan, 2-Butanon, Acetonitril, Sulfan oder Mischungen davon eingesetzt wird.

8. Verfahren nach einem der Ansprüche 4 bis 6, **dadurch gekennzeichnet, dass** der/die gemäß (ii) zugegebenen inerten Stoffe aus den für die Eduktströme eingesetzten Lösungsmitteln ausgewählt sind.

9. Verfahren nach einem der Ansprüche 1 bis 3, **dadurch gekennzeichnet, dass** die Umsetzung in gasförmiger Phase erfolgt.

10. Verfahren nach Anspruch 9, **dadurch gekennzeichnet, dass** der oder die inerten Stoffe aus Inertgasen ausgewählt sind.

11. Verfahren nach Anspruch 9 oder 10, **dadurch gekennzeichnet, dass** das nach der Umsetzung erhaltene Produktgemisch in mindestens einer Quenchzone abgekühlt wird.

12. Verfahren nach einem der Ansprüche 9 bis 11, **dadurch gekennzeichnet, dass** die Umsetzung in Gegenwart mindestens eines Inertmediums durchgeführt wird.

13. Verfahren nach einem der Ansprüche 9 bis 12, **dadurch gekennzeichnet, dass** das Amin ausgewählt ist aus der Gruppe bestehend aus 1,6-Diaminohexan, monomeres 2,4'-Methylen(diphenyldiamin) und/oder monomeres 2,2'-Methylen(diphenyldiamin) und/oder monomeres 4,4'-Methylen(diphenyldiamin), 2,4-Toluylendiamin und/oder 2,6-Toluylendiamin, 1-Amino-3,3,5-trimethyl-5-aminomethylcyclohexan und Mischungen davon.

14. Verfahren gemäß einem der Ansprüche 1 bis 13, **dadurch gekennzeichnet, dass** die Reaktionszone mindestens einen Rohrreaktor umfasst.

## Claims

1. A process for preparing isocyanates by reacting the corresponding amines comprised in at least one feed stream A with phosgene comprised in at least one feed stream P in a reaction plant comprising at least one mixing zone and at least one reaction zone, wherein feed stream A and/or feed stream P optionally comprise one or more inert materials, and
wherein, during periods of time of at least 6h in which the flow S^{x} of the amines used is below 95% of the flow S⁰ of the amines used during operation at the nominal capacity of the reactor plant,
(i) the ratio of phosgene to amine is increased and
(ii) the concentration of the inert material or materials in the amine-comprising feed stream A and/or the phosgene-comprising feed stream P is increased
compared to operation at the nominal capacity of the reactor.

2. The process according to claim 1, wherein the flow of amine is reduced to x % compared to nominal capacity and the total flow G^{x} of amine, phosgene and any inert materials present is at least (x+5)% of the total flow G⁰ of amine, phosgene and any inert materials present at nominal capacity of the plant.

3. The process according to claim 1 or 2, wherein the inert material or materials is/are selected from among inert solvents and inert gases.

4. The process according to any of claims 1 to 3, wherein the reaction is carried out in the liquid phase.

5. The process according to claim 4, wherein the amine is present as solution or as suspension of the corresponding hydrochloride in an inert solvent in the at least one amine-comprising feed stream.

6. The process according to claim 4 or 5, wherein the amine is selected from the group consisting of methylamine, ethylamine, butylamine, stearylamine, phenylamine, p-toluidine, 1,4-diaminobutane, 1,6-diaminohexane, 1,8-diaminooctane, 1,4-diaminobenzene, 2,4- and/or 2,6-diaminotoluene, 2,2'-, 2,4'- and/or 4,4'-diaminodiphenylmethane, alkyl-substituted diamines of the diphenylmethane series, for example 3,4'-diamino-4-methyldiphenylmethane, polyamine mixtures of the diphenylmethane series as are obtained in a known manner by aniline-formaldehyde condensations, p-xylenediamine, perhydrogenated 2,4- and/or 2,6-diaminotoluene, 2,2'-, 2,4'- and/or 4,4'-diaminodicyclohexylmethane, 1-amino-3,3,5-trimethyl-5-aminomethylcyclohexane (isophoronediamine, IPDA for short), the ethyl ester of lysine, the aminoethyl ester of lysine, 2,4- and/or 2,6-toluenediamine, 1,6,11-triaminoundecane and mixtures thereof.

7. The process according to any of claims 4 to 6, wherein monochlorobenzene o-dichlorobenzene, trichlorobenzene, toluene, xylene, methylene chloride, perchloroethylene, trichlorofluoromethane, butyl acetate, hexane, heptane, octane, biphenyl, ethyl acetate, 1,2-diacetoxyethane, 2-butanone, acetonitrile, sulfane or mixtures thereof are used as solvents for the starting materials.

8. The process according to any of claims 4 to 6, wherein the inert material or materials added according to (ii) are selected from among the solvents used for the feed streams.

9. The process according to any of claims 1 to 3, wherein the reaction is carried out in the gaseous phase.

10. The process according to claim 9, wherein the inert material or materials are selected from among inert gases.

11. The process according to claim 9 or 10, wherein the product mixture obtained after the reaction is cooled in at least one quenching zone.

12. The process according to any of claims 9 to 11, wherein the reaction is carried out in the presence of at least one inert medium.

13. The process according to any of claims 9 to 12, wherein the amine is selected from the group consisting of 1,6-diaminohexane, monomeric 2,4'-methylenedi(phenylamine) and/or monomeric 2,2'-methylenedi(phenylamine) and/or monomeric 4,4'-methylenedi(phenylamine), 2,4-toluenediamine and/or 2,6-toluenediamine, 1-amino-3,3,5-trimethyl-5-aminomethylcyclohexane and mixtures thereof.

14. The process according to any of claims 1 to 13, wherein the reaction zone comprises at least one tube reactor.

## Revendications

1. Procédé de fabrication d'isocyanates par réaction des amines correspondantes, contenues dans au moins un flux de réactif A, avec du phosgène, contenu dans au moins un flux de réactif P, dans une installation comportant un réacteur et comprenant au moins une zone de mélange et au moins une zone de réaction, le flux de réactif A et/ou le flux de réactif P contenant éventuellement une ou plusieurs substances inertes, et où, à intervalles d'au moins 6 h, dans lesquels le flux quantitatif S^{x} des amines utilisées est inférieur à 95 % du flux quantitatif S⁰ des amines utilisées lors de l'exploitation à capacité nominale de l'installation comportant un réacteur, en comparaison de l'exploitation à la capacité nominale du réacteur
i) on augmente le rapport du phosgène à l'amine et
ii) on augmente la concentration de la ou des matières inertes dans le flux de réactif A contenant l'amine et/ou dans le flux de réactif P contenant le phosgène.

2. Procédé selon la revendication 1, **caractérisé en ce que** le flux quantitatif d'amine est réduit à x % par rapport à la capacité nominale et le flux quantitatif total G^{x} d'amine, de phosgène et de substances inertes éventuellement présentes est d'au moins (x+5) % du flux quantitatif total G⁰ d'amine, de phosgène et de substances inertes éventuellement présentes à capacité nominale de l'installation.

3. Procédé selon la revendication 1 ou 2, **caractérisé en ce que** le ou les substances inertes sont choisies parmi des solvants inertes et des gaz inertes.

4. Procédé selon l'une des revendications 1 à 3, **caractérisé en ce que** la réaction a lieu en phase liquide.

5. Procédé selon la revendication 4, **caractérisé en ce que** l'amine dans le flux de réactif contenant au moins une amine est présente en solution ou en suspension du chlorhydrate correspondant dans un solvant inerte.

6. Procédé selon la revendication 4 ou 5, **caractérisé en ce que** l'amine est choisie dans le groupe constitué de la méthylamine, éthylamine, butylamine, stéarylamine, phénylamine, p-tolylamine, butan-1,4-diamine, hexan-1,6-diamine, octan-1,8-diamine, 1,4-diaminobenzène, 2,4- et/ou 2,6-diaminotoluène, 2,2'-, 2,4'- et/ou 4,4'-diaminodiphénylméthane, des diamines alkyl-substituées de la série du diphénylméthane, comme par exemple le 3,4'-diamino-4-méthyldiphénylméthane, des mélanges de polyamine de la série du diphénylméthane, comme on en obtient de façon connue par condensations aniline-formaldéhyde, la p-xylènediamine, le 2,4- et/ou 2,6-diaminotoluène perhydrogéné, le 2,2'-, 2,4'- et/ou 4,4'-diaminodicyclohexylméthane, le 1-amino-3,3,5-triméthyl-5-aminométhylcyclohexane (isophoronediamine, abrégé IPDA), le lysinate d'éthyle, le lysinate d'aminoéthyle, la 2,4- et/ou 2,6-toluydènediamine, le 1,6,11-triaminoundécane et leurs mélanges.

7. Procédé selon l'une des revendications 4 à 6, **caractérisé en ce qu'**on utilise comme solvant pour les réactifs du monochlorobenzène, o-dichlorobenzène, trichlorobenzène, toluène, xylène, chlorure de méthylène, perchloréthylène, trichlorofluorométhane, acétate de butyle, hexane, heptane, octane, biphényle, acétate d'éthyle, 1,2-diacétoxyéthane, 2-butanone, acétonitrile, sulfane, ou leurs mélanges.

8. Procédé selon l'une des revendications 4 à 6, **caractérisé en ce que** la ou les substances inertes ajoutées selon ii) sont choisies parmi les solvants utilisés pour les flux de réactifs.

9. Procédé selon l'une des revendications 1 à 3, **caractérisé en ce que** la réaction a lieu en phase gazeuse.

10. Procédé selon la revendication 9, **caractérisé en ce que** la ou les substances inertes sont choisies parmi des gaz inertes.

11. Procédé selon la revendication 9 ou 10, **caractérisé en ce que** le mélange de produits obtenu après la réaction est refroidi dans au moins une zone de refroidissement.

12. Procédé selon l'une des revendications 9 à 11, **caractérisé en ce que** la réaction est effectuée en présence d'au moins un médium inerte.

13. Procédé selon l'une des revendications 9 à 12, **caractérisé en ce que** l'amine est choisie dans le groupe constitué de l'hexan-1,6-diamine, du monomère 2,4'-méthylènediphényldiamine et/ou du monomère 2,2'-méthylènediphényldiamine et/ou du monomère 4,4'-méthylènediphényldiamine, de la 2,4-toluylènediamine et/ou de la 2,6-toluylènediamine, du 1-amino-3,3,5-triméthyl-5-aminométhylcyclohexane et de leurs mélanges.

14. Procédé selon l'une des revendications 1 à 13, **caractérisé en ce que** la zone de réaction comprend au moins un réacteur tubulaire.
